# EUROPEAN PATENT SPECIFICATION

(11) **EP 1 523 300 B1**
(45) Date of publication and mention of the grant of the patent: **03.10.2007**
(21) Application number: 03738110.0
(22) Date of filing: 03.07.2003
(51) Int. Cl.: A61K 8/365, A61Q 5/06

(54) **Hair care method characterised by application of specific 2-hydroxyalkanoic acids**
Methode zur Haarbehandlung gekennzeichnet durch Applikation bestimmter 2-Hydroxyalkansäuren
Procédé de soin des cheveux caractérisé par l'application d'acide 2-hydroxyalcanoïque

(30) Priority: 22.07.2002 EP 02255128
(43) Date of publication of application: 20.04.2005
(73) Proprietor: UNILEVER N.V., 3013 AL Rotterdam (NL); UNILEVER PLC, London EC4P 4BQ (GB)
(72) Inventor: ADAMS, Gerald, Unilever R & D Port Sunlight, Wirral, Merseyside CH63 3JW (GB); KHOSDEL, Ezat, Unilever R & D Port Sunlight, Wirral, Merseyside CH63 3JW (GB); WARD, Sheila Anne, Unilever R & D Port Sunlight, Wirral, Merseyside CH63 3JW (GB)
(74) Representative: Tansley, Sally Elizabeth
(86) International application number: PCT/EP2003/007057
(87) International publication number: WO 2004/009046

(56) References cited:
- EP-A- 0 007 785
- EP-A- 0 403 304
- EP-A- 0 728 778
- WO-A-02/28358
- WO-A-99/15135
- DE-A- 19 536 423
- GB-A- 2 279 359

## Description

### Field of the Invention

This invention relates to uses of hair care compositions, in particular to uses of hair care mousses that style the hair.

### Background and Prior Art

Mousse formulations are frequently applied to damp hair, after shampooing and/or conditioning to style it. Natural or synthetic resins such as polymers of acrylate and polymers of acrylate with grafted silicone are used in the mousse to give the styling benefits. In addition to the styling aid styling mousses frequently comprise a surfactant to form the foam and a propellant.

Shampoo compositions comprising 2-hydroxyalkanoic acids are disclosed in EP 0 403 304 and EP 0 424 158. The 2-hydroxyalkanoic acid is said to enhance the elasticity and the colour penetration respectively.

The present invention has found a way to minimise or obviate the need for a mousse to contain a surfactant/styling polymer/propellant. This means that the mousse is both easy and economical to manufacture.

The mousse to be used in the method of the invention also helps to maintain styled hair from dropping or losing its shape when subjected to high humidity.

### Description of the Present Invention

The invention relates to a method of styling hair by applying to the hair a mousse comprising a 2-hydroxyalkanoic acid - selected from the group consisting of 2-hydroxyhexanoic acid, 2-hydroxy octanoic acid, 2-hyroxydccanoic acid or mixtures thereof, in which the mousse comprises less than 2 wt.% of the total composition of a surfactant.

The invention further relates to the use of a mousse comprising 2-hydroxyalkanoic acid as described above to style and to impart humidity resistance to the hair.

### Detailed Description of the Invention

### 2-hydroxy alkanoic acids

The compositions to be used in the method of the invention comprise as an essential element a 2-hydroxy alkanoic acid selected from the group consisting of 2-hydroxyhexanoic acid, 2-hydroxyoctanoic acid, 2-hydroxydecanoic acid or mixtures thereof. Hydroxyoctanoic acid is especially preferred.

### Styling compound

Particularly useful as styling aids for optional use with this invention are hair styling polymers. Hair styling polymers are well known articles of commerce and many such polymers are available commercially which contain moieties, which render the polymers cationic, anionic, amphoteric or nonionic in nature. The polymers may be synthetic or naturally derived.

Styling aids such as vinylic polymer are preferred, in particular block copolymers.

The amount of the hair styling polymer may range from 0.1 to 10%, preferably 0.5 to 8 %, more preferably 0.75 to 6% by weight based on total weight of the composition.

Examples of anionic hair styling polymers are:
copolymers of vinyl acetate and crotonic acid;
terpolymers of vinyl acetate, crotonic acid and a vinyl ester of an alpha-branched saturated aliphatic monocarboxylic acid such as vinyl neodecanoate;
copolymers of methyl vinyl ether and maleic anhydride (molar ratio about 1:1) wherein such copolymers are 50% esterified with a saturated alcohol containing from 1 to 4 carbon atoms such as ethanol or butanol;
acrylic copolymers containing acrylic acid or methacrylic acid as the anionic radical-containing moiety with other monomers such as: esters of acrylic or methacrylic acid with one or more saturated alcohols having from 1 to 22 carbon atoms (such as methyl methacrylate, ethyl acrylate, ethyl methacrylate, n-butyl acrylate, t-butyl acrylate, t-butyl methacrylate, n-butyl methacrylate, n-hexyl acrylate, n-octyl acrylate, lauryl methacrylate and behenyl acrylate);
glycols having from 1 to 6 carbon atoms (such as hydroxypropyl methacrylate and hydroxyethyl acrylate);
styrene; vinyl caprolactam; vinyl acetate; acrylamide; alkyl acrylamides and methacrylamides having 1 to 8 carbon atoms in the alkyl group (such as methacrylamide, t-butyl acrylamide and n-octyl acrylamide); and other compatible unsaturated monomers.

The polymer may also contain grafted silicone, such as polydimethylsiloxane.

Specific examples of suitable anionic hair styling polymers are:
RESYN® 28-2930 available from National Starch (vinyl acetate/crotonic acid/vinyl neodecanoate copolymer);
ULTRAHOLD® 8 available from BASF (CTFA designation Acrylates/acrylamide copolymer);
the GANTREZ®ES series available from ISP corporation (esterified copolymers of methyl vinyl ether and maleic anhydride).

Other suitable anionic hair styling polymers include carboxylated polyurethanes. Carboxylated polyurethane resins are linear, hydroxyl-terminated copolymers having pendant carboxyl groups. They may be ethoxylated and/or propoxylated at least at one terminal end. The carboxyl group can be a carboxylic acid group or an ester group, wherein the alkyl moiety of the ester group contains one to three carbon atoms. The carboxylated polyurethane resin can also be a copolymer of polyvinylpyrrolidone and a polyurethane, having a CTFA designation PVP/polycarbamyl polyglycol ester. Suitable carboxylated polyurethane resins are disclosed in EP-A-0619111 and US Patent No. 5,000,955. Other suitable hydrophilic polyurethanes are disclosed in US Patent Nos. 3,822,238; 4,156,066; 4,156,067; 4,255,550; and 4,743,673.

Amphoteric hair styling polymers which can contain cationic groups derived from monomers such as t-butyl aminoethyl methacrylate as well as carboxyl groups derived from monomers such as acrylic acid or methacrylic acid can also be used in the present invention. One specific example of an amphoteric hair styling polymer is Amphomer® (Octylacrylamide/ acrylates/butylaminoethyl methacrylate copolymer) sold by the National Starch and Chemical Corporation.

Examples of nonionic hair styling polymers are homopolymers of N- vinylpyrrolidone and copolymers of N-vinylpyrrolidone with compatible nonionic monomers such as vinyl acetate. Nonionic polymers containing N- vinylpyrrolidone in various weight average molecular weights are available commercially from ISP Corporation - specific examples of such materials are homopolymers of N-vinylpyrrolidone having an average molecular weight of about 630,000 sold under the name PVP K-90 and are homopolymers of N-vinylpyrrolidone having an average molecular weight of about 1,000,000 sold under the name of PVP K-120.

Other suitable nonionic hair styling polymers are cross-linked silicone resins or gums. Specific examples include rigid silicone polymers such as those described in EP-A-0240350 and cross-linked silicone gums such as those described in WO 96/31188.

Examples of cationic hair styling polymers are copolymers of amino-functional acrylate monomers such as lower alkyl aminoalkyl acrylate, or methacrylate monomers such as dimethylaminoethyl methacrylate, with compatible monomers such as N-vinylpyrrolidone, vinyl caprolactam, alkyl methacrylates (such as methyl methacrylate and ethyl methacrylate) and alkyl acrylates (such as ethyl acrylate and n-butyl acrylate).

Specific examples of suitable cationic hair styling polymers are:
copolymers of N-vinylpyrrolidone and dimethylaminoethyl methacrylate, available from ISP Corporation as Copolymer 845, Copolymer 937 and Copolymer 958;
copolymers of N-vinylpyrrolidone and dimethylaminopropylacrylamide or methacrylamide, available from ISP Corporation as Styleze® CC10;
copolymers of N-vinylpyrrolidine and dimethylaminoethyl methacrylate;
copolymers of vinylcaprolactam, N-vinylpyrrolidone and dimethylaminoethylmethacrylate;
Polyquaternium-4 (a copolymer of diallyldimonium chloride and hydroxyethylcellulose);
Polyquaternium-11 (formed by the reaction of diethyl sulphate and a copolymer of vinyl pyrrolidone and dimethyl aminoethylmethacrylate), available from ISP as Gafquat® 734, 755 and 755N, and from BASF as Luviquat® PQ11;
Polyquaternium-16 (formed from methylvinylimidazolium chloride and vinylpyrrolidone), available from BASF as Luviquat® FC 370, FC 550, FC 905 and HM-552;
Polyquaternium-46 (prepared by the reaction of vinylcaprolactam and vinylpyrrolidone with methylvinylimidazolium methosulphate), available from BASF as Luviquat®Hold.

Examples of suitable naturally-derived hair styling polymers include shellac, alginates, gelatins, pectins, cellulose derivatives and chitosan or salts and derivatives thereof. Commercially available examples include Kytamer® (ex Amerchol) and Amaze® (ex National Starch).

Also suitable for use as optional components in the compositions of the invention are the ionic copolymers described in WO 93/03703, the polysiloxane-grafted polymers disclosed in WO 93/23446, the silicone-containing polycarboxylic acid copolymers described in WO 95/00106 or WO 95/32703, the thermoplastic elastomeric copolymers described in WO 95/01383, WO 95/06078, WO 95/06079 and WO 95/01384, the silicone grafted adhesive polymers disclosed in WO 95/04518 or WO 95/05800, the silicone macro-grafted copolymers taught in WO 96/21417, the silicone macromers of WO 96/32918, the adhesive polymers of WO 98/48770 or WO 98/48771 or WO 98/48772 or WO 98/48776, the graft polymers of WO 98/51261 and the grafted copolymers described in WO 98/51755.

With certain of the above-described polymers it may be necessary to neutralise some acidic groups to promote solubility/dispersibility. Examples of suitable neutralising agents include 2-amino-2- methyl-1, 3-propanediol (AMPD); 2-amino-2-ethyl-1,3-propanediol (AEPD); 2-amino-2-methyl-1-propanol (AMP); 2-amino-1-butanol (AB); monoethanolamine (MEA); diethanolamine (DEA); triethanolamine (TEA); monoisopropanolamine (MIPA); diisopropanol-amine (DIPA); triisopropanolamine (TIPA); and dimethyl stearamine (DMS). A long chain amine neutralising agent such as stearamidopropyl dimethylamine or lauramidopropyl dimethylamine may be employed, as is described in US 4,874,604. Also suitable are inorganic neutralisers, examples of which include sodium hydroxide, potassium hydroxide and borax. Mixtures of any of the above neutralising agents may be used. Amounts of the neutralising agents will range from about 0.001 to about 10% by weight of the total composition.

### Surfactants

It is preferable if surfactant is absent from the formulation. If present only low levels of surfactant are used at levels below 2 wt%.

The surfactant, if present, is preferably selected from anionic, nonionic, amphoteric and zwitterionic surfactants, and mixtures thereof.

Suitable anionic surfactants include the alkyl sulphates, alkyl ether sulphates, alkaryl sulphonates, alkanoyl isethionates, alkyl succinates, alkyl sulphosuccinates, N-alkoyl sarcosinates, alkyl phosphates, alkyl ether phosphates, alkyl ether carboxylates, and alpha-olefin sulphonates, especially their sodium, magnesium, ammonium and mono-, di- and triethanolamine salts.

The most preferred anionic surfactants are sodium lauryl sulphate, triethanolamine lauryl sulphate, triethanolamine monolauryl phosphate, sodium lauryl ether sulphate 1EO, 2EO and 3EO, ammonium lauryl sulphate and ammonium lauryl ether sulphate 1EO, 2EO and 3EO.

Nonionic surfactants suitable for use in compositions of the invention may include condensation products of aliphatic (C₈-C₁₈) primary or secondary linear or branched chain alcohols or phenols with alkylene oxides, usually ethylene oxide and generally having from 6 to 30 ethylene oxide groups. Other suitable nonionics include mono- or di-alkyl alkanolamides. Example include coco mono- or di- ethanolamide and coco mono-isopropanolamide.

Further nonionic surfactants which can be included in shampoos for the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)ₙ

wherein R is a branched or straight chain alkyl group which may be saturated or unsaturated and G is a saccharide group. Suitable alkyl polyglycosides for use in the invention are commercially available and include for example those materials identified as: Oramix NS10 ex Seppic; Plantaren 1200 and Plantaren 2000 ex Henkel.

Amphoteric and zwitterionic surfactants suitable for use in compositions of the invention may include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Examples include lauryl amine oxide, cocodimethyl sulphopropyl betaine and preferably lauryl betaine, cocamidopropyl betaine and sodium cocamphopropionate.

### Propellants

In some instances mousses of the invention comprise a propellant.

Examples of suitable propellants include materials such as trichlorofluoromethane, dichlorodifluoromethane, difluoroethane, dimethylether, propane, n-butane or isobutane.

The level of propellant can be adjusted as desired but is generally from about 3% to about 25% by weight based on total weight for mousse compositions and from about 10% to 20%.

### Further components

The mousses of the invention may include a carrier and further additional components. The carriers and additional components required to formulate such products vary with product type and can be routinely chosen by one skilled in the art. The following is a description of some of these carriers and additional components.

Solvents include water, ethanol, volatile silicone derivatives, and mixtures thereof. The solvents used in such mixtures may be miscible or immiscible with each other. The mousses may also utilise any of the conventional propellants listed above to deliver the material as a foam. An emulsifying agent may be present. Examples of suitable emulsifying agents include nonionic, cationic, anionic surfactants, or mixtures thereof. If such an emulsifying agent is used, it is preferably present at a level of from about 0.01% to about 7.5% by weight based on total weight of the composition.

The mousse can include a wide variety of conditioning materials such as cationic conditioners suitable for hair, quaternary silicone polymers, silicone based conditioners and their emulsions, and amino functional silicones and their emulsions.

Further general ingredients suitable for all product forms include, sun-screening agents, anti-dandruff actives, carboxylic acid polymer thickeners for hair shampoo and conditioner compositions and emulsifiers for emulsifying the various carrier components of the compositions of the invention.

The compositions of the present invention may also contain adjuncts suitable for hair care. Generally such ingredients are included individually at a level of up to 2, preferably up to 1 wt% of the total composition. Suitable hair care adjuncts, include amino acids, sugars and ceramides.

### Packaging

Suitable spray containers are well known in the art and include conventional, non-aerosol pump sprays i.e., "atomisers", aerosol containers or cans having propellant, as described above, and also pump aerosol containers utilising compressed air as the propellant.

The method of the invention comprises applying the mousse to the hair during or immediately before styling. It is preferable if the mousse is applied to wet or damp hair.

The following non-limiting Examples further illustrate the preferred embodiments of the invention. All percentages referred to in the examples and throughout this specification are by weight based on total weight unless otherwise indicated.

### EXAMPLES

### Examples 1 to 2 :

### Example 1

A styling mousse is formulated as follows:

| **Material** | **% in product (w/w)** |
|---|---|
| Silicone Emulsion X2 1787 (Dow Corning) | 1.2 |
| Hydroxyoctanoic acid | 1.5 |
| Gafquat 734 (Polyquaternium-11 ISP) | 0.3 |
| VOLPO CS 50 (Croda Chemicals) | 0.1 |
| Sepicide LD | 0.4 |
| Cremophor RH410 | 0.2 |
| Ethanol | 7.5 |
| CAP 40 | 6.0 |
| Perfume | 0.2 |
| Water | to 100% |

### Example 2

### Wiglet test procedure

To ensure equal dosage of active, 10g of test compound 2 was weighed and applied to the left-hand side of the wiglet and 2.5g of test compound 1 applied to the right-hand side of the wiglet. This was left on the hair for 20 minutes and then set in rollers and dried under a hood dryer for 30 minutes to set the style. The rollers are carefully removed and the initial style photographed. Photographs are taken throughout the study. The style retention is monitored by the stylist over a 30 minute time period under a controlled environment of 30°C and 90%RH using the Sanyo Gallenkamp Chamber.

The following formulations were prepared and tested on wiglets:

| Experiment | Materials | Weight Percent | Solvent | Stylists' comments |
|---|---|---|---|---|
| Negative Control | Water | 100% | Water | Very poor style retention Curls collapsed quickly and style is 'messy' |
| Test Compound 1 Propellant mousse can | 2 hydroxy octanoic acid | 4% | Water | Good style retention. Curls are still in good shape Looks shiny and clean |
| | | 8% | | |
| | CAP 40 | | | |
| Test Compound 2 Non propellant based mousse can | 2 hydroxy octanoic acid | 1% | Water | Also good style retention but curls have opened slightly more than right hand side |

## Claims

1. A method of styling hair by applying to the hair a hair treatment mousse comprising a 2-hydroxyalkanoic acid selected from the group consisting of 2-hydroxyhexanoic acid, 2-hydroxyoctanoic acid, 2-hydroxydecanoic acid or mixtures thereof, in which the mousse comprises less than 2-wt.% of the total composition of a surfactant.

2. A method of styling hair according to claim 1 in which the 2-hydroxyalkanoic acid is 2-hydroxyoctanoic acid.

3. A method of styling hair according to claim 1 or claim 2 in which the level of 2-hydroxyalkanoic acid within the mousse is from 0.5 to 10 wt%.

4. A method of styling hair according to any preceding claim wherein the mousse further comprising a styling polymer.

5. A method of styling hair according to any preceding claim, wherein the mousse further comprising a propellant.

6. Use of a mousse described in any case of claims 1 to 5 to style hair.

7. Use of a mousse described in any one of claims 1 to 5 to impart humidity resistance to hair.

## Patentansprüche

1. Verfahren zum Haarstyling durch Auftragen auf das Haar einer Haarbehandlungsmousse, die 2-Hydroxyalkansäure, ausgewählt aus der Gruppe, bestehend aus 2-Hydroxyhexansäure, 2-Hydroxyoctansäure, 2-Hydroxydecansäure oder Gemischen davon, umfasst, wobei die Mousse weniger als 2 Gewichtsprozent der Gesamtzusammensetzung von einem Tensid umfasst.

2. Verfahren zum Haarstyling nach Anspruch 1, wobei die 2-Hydroxyalkansäure 2-Hydroxyoctansäure ist.

3. Verfahren zum Haarstyling nach Anspruch 1 oder Anspruch 2, wobei der Anteil von 2-Hydroxyalkansäure in der Mousse 0,5 bis 10 Gewichtsprozent ist.

4. Verfahren zum Haarstyling nach einem vorangehenden Anspruch, wobei die Mousse weiterhin ein Stylingpolymer umfasst.

5. Verfahren zum Haarstyling nach einem vorangehenden Anspruch, wobei die Mousse weiterhin ein Treibmittel umfasst.

6. Verwendung einer in jedem Fall von Ansprüchen 1 bis 5 beschriebenen Mousse zum Haarstyling.

7. Verwendung einer wie in einem der Ansprüche 1 bis 5 beschriebenen Mousse, um dem Haar Feuchtigkeitsbeständigkeit zu verleihen.

## Revendications

1. Méthode de coiffage par application sur les cheveux d'une mousse de traitement des cheveux comprenant un acide 2-hydroxyhexanoique choisi dans le groupe consistant en l'acide 2-hydroxyhexanoïque, l'acide 2-hydroxyoctanoïque, l'acide 2- hydroxydécanoïque ou des mélanges de ceux-ci, dans laquelle la mousse comprend moins de 2 % en poids par rapport à la composition totale d'un tensioactif.

2. Méthode de coiffage selon la revendication 1 dans laquelle l'acide 2-hydroxyalcanoique est l'acide 2-hydroxyoctanoïque.

3. Méthode de coiffage selon la revendication 1 ou la revendication 2 dans laquelle le niveau d'acide 2- hydroxyalcanoïque dans la mousse est de 0,5 à 10 % en poids.

4. Méthode de coiffage selon l'une quelconque des revendications précédentes dans laquelle la mousse comprend en outre un polymère coiffant.

5. Méthode de coiffage selon l'une quelconque des revendications précédentes dans laquelle la mousse comprend en outre un propulseur.

6. Utilisation d'une mousse décrite dans l'une quelconque des revendications 1 à 5 destinée au coiffage.

7. Utilisation d'une mousse décrite dans l'une quelconque des revendications 1 à 5 destinée à impartir aux cheveux de la résistance à l'humidité.
